(19) Europäisches Patentamt
European Patent Office
Office européen des brevets

(11) Veröffentlichungsnummer: **0 307 791**
**A1**

(12) **EUROPÄISCHE PATENTANMELDUNG**

(21) Anmeldenummer: **88114655.9**

(22) Anmeldetag: **08.09.88**

(51) Int. Cl.4: **C07D 249/08 , C07D 233/60 , A01N 43/653 , A01N 43/50**

(30) Priorität: **15.09.87 DE 3730871**

(43) Veröffentlichungstag der Anmeldung: **22.03.89 Patentblatt 89/12**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Holmwood, Graham, Dr.**
**Krutscheider Weg 105**
**D-5600 Wuppertal 11(DE)**
Erfinder: **Krämer, Wolfgang, Dr.**
**Rosenkranz 25**
**D-5093 Burscheid 2(DE)**
Erfinder: **Regel, Erik, Dipl.-Ing.**
**Untere Bergerheide 26**
**D-5600 Wuppertal(DE)**
Erfinder: **Elbe, Hans-Ludwig, Dr.**
**Dasnöckel 59**
**D-5600 Wuppertal 11(DE)**
Erfinder: **Büchel, Karl-Heinz, Prof. Dr.**
**Dabringhauser Strasse 42**
**D-50993 Burscheid(DE)**
Erfinder: **Dutzmann, Stefan, Dr.**
**Leinenweberweg 33**
**D-4000 Düsseldorf 13(DE)**
Erfinder: **Brandes, Wilhelm, Dr.**
**Eichendorffstrasse 3**
**D-5653 Leichlingen(DE)**
Erfinder: **Reinecke, Paul, Dr.**
**Steinstrasse 8**
**D-5090 Leverkusen 3(DE)**

(54) **Bisazolyl-hydroxyalkyl-Derivate.**

(57) Neue Bisazolyl-hydroxyalkyl-Derivate der Formel

EP 0 307 791 A1

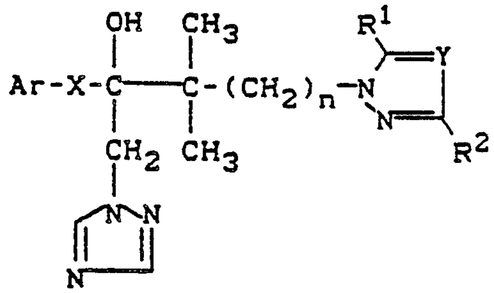

(I)

in welcher

Ar für gegebenenfalls substituiertes Aryl steht,

X für eine Gruppierung $-CH_2CH_2-$, $-OCH_2-$, $-SCH_2-$ oder $-CH=CH-$ steht,

Y für ein Stickstoffatom oder die Gruppierung $CR^3$ steht,

$R^1$, $R^2$ und $R^3$ für Wasserstoff, Halogen oder Methyl stehen und

n für die Zahlen 0, 1 oder 2 steht,

sowie deren Säureadditions-Salze und Metallsalz-Komplexe, ein Verfahren zur Herstellung der neuen Stoffe und deren Verwendung als Fungizide.

Neue Zwischenprodukte, Verfahren zu deren Herstellung und deren Verwendung zur Synthese von Bisazolyl-hydroxyalkyl-Derivaten der Formel (I).

## Bisazolyl-hydroxyalkyl-Derivate

Die vorliegende Erfindung betrifft neue Bisazolyl-hydroxyalkyl-Derivate, ein Verfahren zu ihrer Herstellung sowie ihre Verwendung als Fungizide.

Es ist bereits bekannt, daß zahlreiche substituierte Triazolylalkyl-triazolylmethyl-carbinole fungizide Eigenschaften besitzen (vergl. EP-OS 0 044 605 und EP-OS 0 131 845). Die Wirkung dieser Verbindungen ist jedoch vor allem bei niedrigen Aufwandmengen und -konzentrationen nicht immer ganz zufriedenstellend.

Es wurden nun neue Bisazolyl-hydroxyalkyl-Derivate der Formel

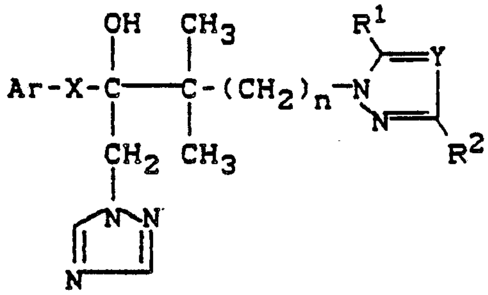

(I)

in welcher

Ar für gegebenenfalls substituiertes Aryl steht,

X für eine Gruppierung $-CH_2CH_2-$, $-OCH_2-$, $-SCH_2-$ oder $-CH=CH-$ steht,

Y für ein Stickstoffatom oder die Gruppierung $CR^3$ steht,

$R^1$, $R^2$ und $R^3$ für Wasserstoff, Halogen oder Methyl stehen und

n für die Zahlen 0, 1 oder 2 steht,

sowie deren Säureadditions-Salze und Metallsalz-Komplexe gefunden.

Die neuen Bisazolyl-hydroxyalkyl-Derivate der Formel (I) besitzen ein asymmetrisch substituiertes Kohlenstoffatom und können deshalb in den beiden optischen Isomerenformen anfallen. Die Erfindung betrifft sowohl die Racemate als auch die einzelnen Isomeren und deren Gemische.

Außerdem können die erfindungsgemäßen Verbindungen der Formal (I), in denen X für $-CH=CH-$ steht, in den geometrischen Isomerenformen cis und trans vorkommen. Die Erfindung betrifft sowohl die einzelnen Isomeren als auch deren Gemische.

Weiterhin wurde gefunden, daß man Bisazolyl-hydroxyalkyl-Derivate der Formel (I) sowie deren Säureadditions-Salz und Metallsalz-Komplexe erhält, wenn man Oxirane der Formel

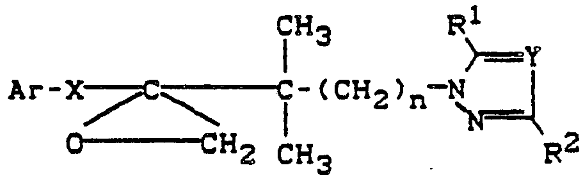

(II)

in welcher

Ar, X, Y, $R^1$, $R^2$ und n die oben angegebene Bedeutung haben,

mit 1,2,4-Triazol der Formel

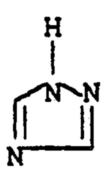

(III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

Schließlich wurde gefunden, daß sich die Bisazolylhydroxyalkyl-Derivate der Formel (I) sowie deren Säureadditions-Salze und Metallsalz-Komplexe durch sehr gute fungizide Eigenschaften auszeichnen.

Überraschenderweise besitzen die erfindungsgemäßen Stoffe eine bessere fungizide Wirksamkeit als aus dem Stand der Technik bekannte substituierte Triazolylalkyltriazolylmethyl-carbinole, welches konstitutionell ähnliche Wirkstoffe gleicher Wirkungsrichtung sind.

Die erfindungsgemäßen Bisazolyl-hydroxyalkyl-Derivate sind durch die Formel (I) allgemein definiert. Bevorzugt sind diejenigen Verbindungen der Formel (I), in denen

Ar für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 oder 2 Kohlenstoffatomen, Nitro, Halogenalkyl, Halogenalkoxy und Halogenalkylthio mit jeweils 1 oder 2 Kohlenstoffatomen und 1 bis 5 gleichen oder verschiedenen Halogenatomen, wie vorzugsweise Fluor- und Chloratomen, Alkoximinoalkyl mit 1 bis 4 Kohlenstoffatomen im Alkoxyteil und 1 oder 2 Kohlenstoffatomen im Alkylteil sowie jeweils gegebenenfalls durch Halogen und/oder Alkyl mit 1 oder 2 Kohlenstoffatomen substituiertes Phenyl, Phenoxy, Benzyl und Benzyloxy;

Ar außerdem für gegebenenfalls einfach bis dreifach, gleich oder verschieden substituiertes Naphthyl steht, wobei als Substituenten genannt seien: Halogen, Alkyl und Alkoxy mit jeweils 1 oder 2 Kohlenstoffatomen sowie Halogenalkoxy mit 1 oder 2 Kohlenstoffatomen und 1 bis 5 gleichen oder verschiedenen Halogenatomen, wie vorzugsweise Fluor-und Chloratomen;

X für die Gruppierungen $-CH_2CH_2-$, $-OCH_2-$, $-SCH_2-$ oder $-CH=CH-$ steht, wobei das Heteroatom mit dem Aryl-Rest verbunden ist, wenn X für $-OCH_2-$ oder $-SCH_2-$ steht;

Y für ein Stickstoffatom oder die Gruppierung $CR^3$ steht;

$R^1$, $R^2$ und $R^3$ für Wasserstoff, Chlor, Brom oder Methyl stehen und

n für die Zahlen 0, 1 oder 2 steht.

Besonders bevorzugt sind diejenigen Verbindungen der Formel (I), in denen

Ar für gegebenenfalls einfach bis dreifach, gleich oder verschieden substituiertes Phenyl steht, wobei als Substituenten genannt seien: Fluor, Chlor, Brom, Jod, Methyl, Methoxy, Methylthio, Nitro, Trifluormethyl, Trifluormethoxy, Trifluormethylthio, Methoximinomethyl, Methoximinoethyl, Ethoximinomethyl sowie jeweils gegebenenfalls durch Fluor, Chlor und/oder Methyl substituiertes Phenyl, Phenoxy, Benzyl und Benzyloxy; oder

Ar für Naphthyl steht;

X für die Gruppierungen $-CH_2CH_2-$, $-OCH_2-$, $-SCH_2-$ oder $-CH=CH-$ steht, wobei das Heteroatom mit dem Aryl-Rest verbunden ist, wenn X für $-OCH_2-$ oder $-SCH_2-$ steht;

Y für ein Stickstoffatom oder die Gruppierung $CR^3$ steht;

$R^1$, $R^2$ und $R^3$ für Wasserstoff, Chlor oder Methyl stehen und

n für die Zahlen 0, 1 oder 2 steht.

Bevorzugte erfindungsgemäße Verbindungen sind auch Additionsprodukte aus Säuren und denjenigen Bisazolyl-hydroxyalkyl-Derivaten der Formel (I), in denen Ar, X, Y, $R^1$, $R^2$ und n diejenigen Bedeutungen haben, die bereits im Zusammenhang mit der Beschreibung der erfindungsgemäßen Stoffe für diese Substituenten bzw. diesen Index als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Bisazolyl-hydroxyalkyl-Derivaten der Formel (I), in denen Ar, X, Y, $R^1$, $R^2$ und n diejenigen Bedeutungen haben, die bereits im Zusammenhang mit der Beschreibung der erfindungsgemäßen Stoffe für diese Substituenten bzw. diesen Index als bevorzugt genannt wurden.

Hierbei sind Salze des Kupfers, Zinks, Mangans, Magnesiums, Zinks, Eisens und des Nickels besonders bevorzugt. Als Anionen dieser Salze kommen solche in Betracht, die sich von solchen Säuren ableiten, die zu pflanzenverträglichen ·additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure.

Verwendet man beispielsweise 2-(4-Chlorphenylethyl)-2-(pyrazol-1-yl-prop-2-yl)-oxiran und 1,2,4-Triazol

als Ausgangsstoffe, so kann der Verlauf des erfindungsgemäßen Verfahrens durch das folgende Formelschema wiedergegeben werden:

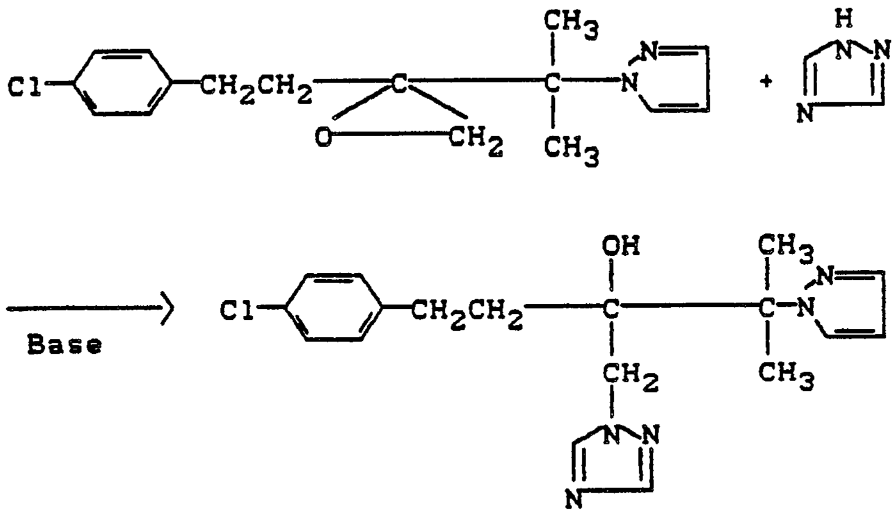


Die bei der Durchführung des erfindungsgemäßen Verfahrens als Ausgangsstoffe zu verwendenden Oxirane sind durch die Formel (II) allgemein definiert. In dieser Formel haben Ar, X, Y, $R^1$, $R^2$ und der Index n vorzugsweise die Bedeutungen, die bereits im Zusammenhang mit der Beschreibung der erfindungsgemäßen Stoffe der Formel (I) für diese Substituenten bzw. für den Index als bevorzugt genannt wurden.

Die Oxirane der Formel (II) sind noch nicht bekannt. Sie lassen sich herstellen, indem man

a) in einer ersten Stufe Ketone der Formel

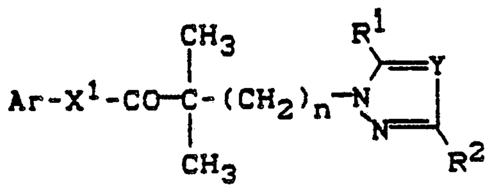
(IVa)

in welcher
Ar, Y, $R^1$, $R^2$ und n die oben angegebene Bedeutung haben und
$X^1$ für die Gruppierungen $-CH_2CH_2-$, $-OCH_2$ und $-SCH_2-$ steht,
mit Methyl-triphenyl-phosphoniumbromid der Formel

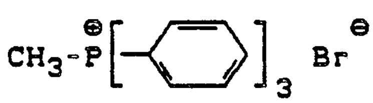
(V)

in Gegenwart einer Base und in Gegenwart eines Verdünnungsmittels umsetzt und dann in einer zweiten Stufe die so erhaltenen Verbindungen der Formel

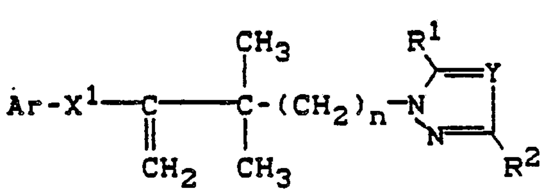
(VI)

in welcher
Ar, $X^1$, Y, $R^1$, $R^2$ und n die oben angegebene Bedeutung haben,
mit Persäure in Gegenwart eines Verdünnungsmittels umsetzt;

oder

(b) Ketone der Formel

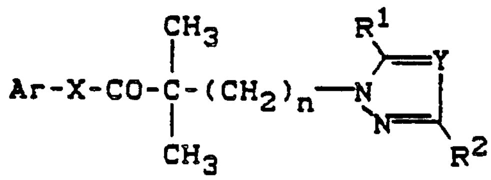

(IV)

in welcher Ar, X, Y, $R^1$, $R^2$ und n die oben angegebene Bedeutung haben, entweder

α) mit Dimethyloxosulfonium-methylid der Formel

$$\overset{\delta\oplus}{(CH_3)_2SO}\ \overset{\delta\ominus}{CH_2} \qquad (VII)$$

oder

β) mit Dimethylsulfonium-methylid der Formel

$$\overset{\delta\oplus}{(CH_3)_2S}\ \overset{\delta\ominus}{CH_2} \qquad (VIII)$$

in Gegenwart eines Verdünnungsmittels umsetst.

Die bei der Herstellung der Oxirane der Formel (II) als Ausgangsstoffe benötigten Ketone der Formel (IV) bzw. (IVa) sind noch nicht bekannt. Sie lassen sich nach im Prinzip bekannten Verfahren herstellen (vgl. z.B. EP-OS 0 084 834). So erhält man Ketone der Formel

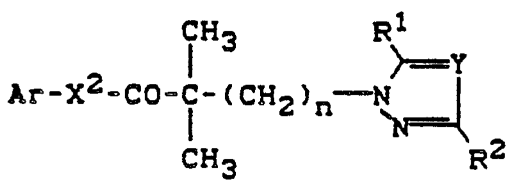

(IVb)

in welcher Ar, Y, $R^1$, $R^2$ und n die oben angegebene Bedeutung haben und

$X^2$ für Gruppierungen $-OCH_2-$ und $-SCH_2-$steht,

indem man Halogenketone der Formel

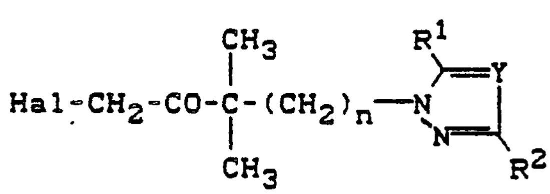

(IX)

in welcher
Y, $R^1$, $R^2$ und n die oben angegebene Bedeutung haben und
Hal für Chlor oder Brom steht,
mit Verbindungen der Formel

Ar-Z-H (X)

in welcher
Ar die oben angegebene Bedeutung hat und
Z für Sauerstoff oder Schwefel steht,
in Gegenwart einer Base, wie z.B. Kaliumcarbonat oder Triethylamin und in Gegenwart eines Verdünnungsmittels, wie z.B. Aceton oder Acetonitril, bei Temperaturen zwischen 20°C und 100°C umsetzt.

Weiterhin erhält man Ketone der Formel

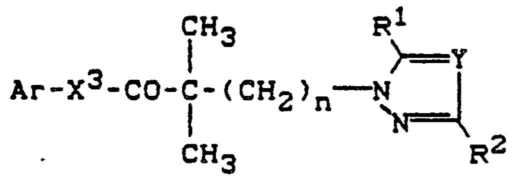

(IVc)

in welcher
Ar, Y, $R^1$, $R^2$ und n die oben angegebene Bedeutung haben und
$X^3$ für die Gruppierungen $-CH_2CH_2-$ und -CH=CH- steht, in dem man Verbindungen der Formel

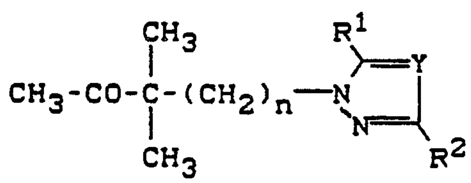

(XI)

in welcher
Y, $R^1$, $R^2$ und n die oben angegebene Bedeutung haben mit Aldehyden der Formel

Ar-CHO (XII)

in welcher
Ar die oben angegebene Bedeutung hat,
in Gegenwart eines Verdünnungsmittels, wie z.B. Ethanol, und in Gegenwart einer Base, wie z.B. Natriumhydroxid, bei Temperaturen zwischen 0 und 60°C umsetzt und gegebenenfalls die entstehenden Verbindungen der Formel

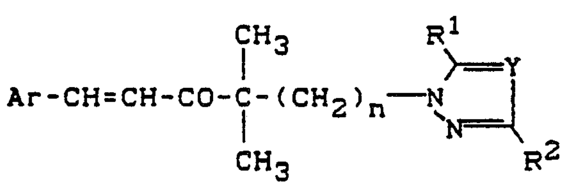

(IVd)

in welcher
Ar, $R^1$, $R^2$, Y und n die oben angegebene Bedeutung haben,
mit Wasserstoff in Gegenwart eines Katalysators, wie z.B. Raney-Nickel, in Gegenwart eines Verdünnungsmit tels, wie z.B. Methanol, Toluol oder Tetrahydrofuran, bei Temperaturen zwischen 40 und 180°C hydriert (vgl. auch die Herstellungsbeispiele).

In manchen Fällen erweist es sich als vorteilhaft, neben der -CH=CH-Doppelbindung gleichzeitig die Keto-Gruppe mitzuhydrieren und diese anschließend in üblicher Art und Weise, wie z.B. mit

Chromsäure/Eisessig, wieder zu oxidieren (vgl. auch die Herstellungsbeispiele).

Die Halogenketone der Formel (IX) werden in bekannter Art und Weise durch übliche Halogenierung der Verbindungen der Formel (XI) erhalten.

Die Verbindungen der Formel (XI) werden in bekannter Art und Weise erhalten, indem man Verbindungen der Formel

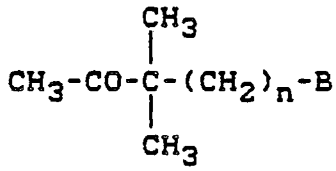

(XIII)

in welcher

B für eine übliche Abgangsgruppe, wie Chlor, Brom, Methylsulfonyloxy oder p-Methylphenylsulfonyloxy steht und

n die oben angegebene Bedeutung hat mit Azolen der Formel

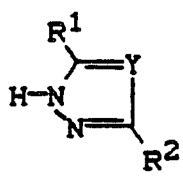

(XIV)

in welcher

Y, $R^1$ und $R^2$ die oben angegebene Bedeutung haben, in der Schmelze oder gegebenenfalls in Gegenwart eines Lösungsmittels, wie z.B. Aceton, und in Gegenwart eines Säurebindemittels, wie z.B. Kaliumcarbonat, bei Temperaturen zwischen 60 und 120 °C umsetzt. Die Azole der Formel (XIV) können aber auch in Form ihrer Alkalisalze, die in üblicher Weise in-situ hergestellt werden, eingesetzt werden.

Sowohl die Verbindungen der Formel (X) und (XIII) als auch die Aldehyde der Formel (XIII) und die Azole der Formel (XIV) sind bekannt oder lassen sich nach bekannten Verfahren herstellen.

Das bei der Herstellung der Oxirane der Formel (II) nach dem Verfahren (a) weiterhin als Ausgangsmaterial benötigte Methyl-triphenyl-phosphonium-bromid der Formel (V) ist bekannt.

Die bei der Herstellung der Oxirane der Formel (II) nach dem obigen Verfahren (a) in der zweiten Stufe als Ausgangssubstanzen benötigten Verbindungen der Formel (VI) sind bisher noch nicht bekannt.

Bei dem Verfahren (a) zur Herstellung der Oxirane der Formel (II) arbeitet man in der ersten Stufe in Gegenwart einer Base. Dabei kommen als Basen alle üblicherweise für Wittig-Reaktionen dieser Art verwendbaren Basen in Betracht. Vorzugsweise verwendbar ist Kaliumtert.-butylat.

Als Verdünnungsmittel kommen bei der Durchführung der ersten Stufe des obigen Verfahrens (a) zur Herstellung der Oxirane der Formel (II) alle für derartige Umsetzungen üblichen organischen Solventien infrage. Vorzugsweise verwendbar sind aromatische Kohlenwasserstoffe, wie Benzol, Toluol und Xylol.

Bei der Durchführung der zweiten Stufe des obigen Verfahrens (a) zur Herstellung der Oxirane der Formel (II) kommen als Reagenzien zur Epoxidierung alle üblichen Persäuren in Betracht. Vorzugsweise verwendbar sind meta-Chlorperbenzoesäure und Peressigsäure. Ferner ist es auch möglich, ein Gemisch aus Essigsäure und Wasserstoffperoxid einzusetzen.

Als Verdünnungsmittel kommen bei der Durchführung der zweiten Stufe des obigen Verfahrens (a) zur Herstellung der Oxirane der Formel (II) alle für derartige Epoxidierungen üblichen Solventien infrage. Vorzugsweise verwendbar sind Dichlormethan, Chloroform, Toluol, Dichlorbenzol, Essigsäure und andere inerte Solventien.

Die Reaktionstemperaturen können bei der Durchführung des Verfahrens (a) zur Herstellung der Oxirane der Formel (II) innerhalb eines bestimmten Bereiches variiert werden. Im allgemeinen arbeitet man in der ersten Stufe bei Temperaturen zwischen 50 °C und 140 °C, vorzugsweise zwischen 80 °C und 120 °C. In der zweiten Stufe arbeitet man im allgemeinen zwischen 10 °C und 60 °C, vorzugsweise zwischen 20 °C und 50 °C.

Bei der Durchführung des Verfahrens (a) zur Herstellung der Oxirane der Formel (II) geht man im allgemeinen so vor, daß man in der ersten Stufe auf 1 Mol an Keton der formel (IVa) zwischen 1 und 3 Mol

Methyl-triphenylphosphonium-bromid der Formel (V) sowie zwischen 1 und 3 Mol Base einsetzt. In der zweiten Stufe setzt man auf 1 Mol an einer Verbindung der Formel (VI) jeweils zwischen 1 und 2 Mol an Persäure ein. Die Aufarbeitung erfolgt jeweils nach üblichen Methoden.

Das bei dem Verfahren (b) als Reaktionskomponente benötigte Dimethyloxosulfoniummethylid der formel (VII) ist bekannt (vgl. J. Amer. Chem. Soc. 87, 1363-1364 (1965)). Es wird bei der obigen Umsetzung in frisch zubereitetem Zustand verarbeitet, indem man es in situ durch Umsetzung von Trimethyloxosulfonium-iodid mit Natriumhydrid oder Natriumamid, insbesondere mit Kalium-tert.-butylat oder Natriummethylat, in Gegenwart eines Verdünnungsmittels erzeugt.

Das bei dem Verfahren (b) außerdem als Reaktionskomponente in Betracht kommende Dimethylsulfonium-methylid der Formel (VIII) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfonium-methylsulfat, in Gegenwart einer starken Base, wie zum Beispiel Natriumhydrid, Natriumamid, Natriummethylat, Kaliumtert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (b) inerte organische Solventien infrage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie dimethylsulfoxid.

Die Reaktionstemperaturen können bei dem Verfahren (b) in einem größeren Bereich variiert werden. Im allgemeinen arbeitet man bei Temperaturen zwischen 0 und 100°C, vorzugsweise zwischen 10 und 60°C.

Bei der Durchführung des Verfahrens (b) setzt man auf 1 Mol an Keton der Formel (IV), vorzugsweise 1 bis 3 Mol an Dimethyloxosulfonium-methylid der Formel (VII) bzw. an Dimethylsulfonium-methylid der formel (VIII) ein. Die Isolierung der Oxirane erfolgt nach üblichen Methoden.

Die Oxirane der Formel (II) können bei dem erfindungsgemäßen Verfahren gegebenenfalls ohne Isolierung direkt weiter umgesetzt werden.

Als Verdünnungsmittel kommen für das erfindungsgemäße Verfahren unter den Reaktionsbedingungen inerte organische Lösungsmittel infrage. Vorzugsweise verwendbar sind Alkohole, wie Ethanol, Methoxyethanol oder Propanol; Keton, wie 2-Butanon und N-Methyl-pyrrolidon; Nitrile, wie Acetonitril; Ester, wie Essigester; Ether, wie Dioxan; aromatische Kohlenwasserstoffe, wie Benzol und Toluol; oder Amide, wie Dimethylformamid.

Als Basen kommen für das erfindungsgemäße Verfahren alle üblicherweise verwendbaren anorganischen und organischen Basen in Betracht. Vorzugsweise verwendbar sind Alkalimetallcarbonate, wie Natrium- und Kaliumcarbonat; Alkalimetallhydroxide, wie Natriumhydroxid; Alkalimetallalkoholate, wie Natrium- und Kalium-methylat und -ethylat; Alkalimetallhydride, wie Natriumhydrid; sowie niedere tertiäre Alkylamine, Cycloalkylamine und Aralkylamine, wie insbesondere Triethylamin.

Als Katalysatoren kommen bei der Durchführung des erfindungsgemäßen Verfahrens alle üblicherweise für derartige Umsetzungen einsetzbaren Reaktionsbeschleuniger infrage. Vorzugsweise verwendbar ist $\alpha,\alpha'$-Azo-isobutyronitril.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Bei der Durchführung des erfindungsgemäßen Verfahrens setzt man auf 1 Mol Oxiran der Formel (II) vorzugsweise 1 bis 1 Mol an 1,2,4-Triazol und gegebenenfalls 1 bis 2 Mol an Säurebindemittel ein. Die Aufarbeitung und die Isolierung der Endprodukte erfolgt nach üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen

vorzugsweise diejenigen Salze von Metallen infrage, die bereits weiter oben beschrieben wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Stoffe mit besonders gutem Erfolg zur Bekämpfung von Venturia (Apfel), Sphaerotheca (Gurke) sowie von Erysiphe, Leptosphaeria, Cochliobolus, Pyrenophora, Pseudocercosporella und Cercospora an Getreide, von Pyricularia an Reis sowie von Rostpilzen, insbesondere in Getreide, eingesetzt werden. Darüberhinaus besitzen die erfindungsgemäßen Verbindungen ein gutes, breites in-vitro-Wrikungsspektrum.

In entsprechenden Aufwandmengen zeigen die erfindungsgemäßen Stoffe auch eine pflanzenwachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Steckmitteln, als flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als

flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farb stoffe, wie Alizarin-, Azo- und Metallphthalocyaninfarbstoffe und Spurennährstoffe wie salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Die Herstellung und die Verwendung der erfindungsgemäßen Stoffe geht aus den folgenden Beispielen hervor.

<u>Herstellungsbeispiele:</u>

Beispiel 1

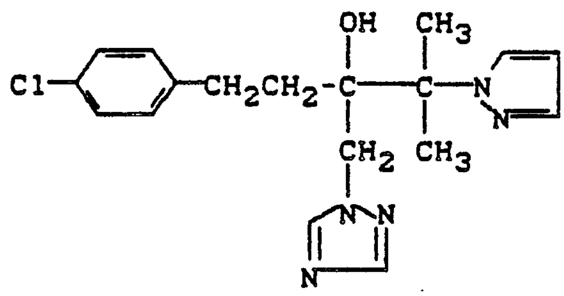

(I-1)

Zu einer Lösung von 26 g (0,08 Mol) 2-(4-Chlorphenylethyl)-2-(pyrazol-1-yl-prop-2-yl)-oxiran in 150 ml n-Butanol werden 11 g (0,16 Mol) 1,2,4-Triazol und 0,9 g (0,016 Mol) gepulvertes Kaliumhydroxid gegeben. Die Mischung wird 10 Stunden bei 120 °C gerührt. Danach wird das Lösungsmittel unter vermindertem Druck abdestilliert. Der verbleibende Rückstand wird in 200 ml Methylenchlorid aufgenommen, die organische Phase wird dreimal mit je 150 ml Wasser gewaschen, über Natriumsulfat getrocknet und anschließend unter vermindertem Druck eingeengt. Man erhält 24 g einer oeligen Substanz, die durch Säurenchromatographie mit Methylenchlorid an Kieselgel 60 Merck gereinigt wird. Nach Umkristallisation des isolierten Produktes erhält man 4,6 g (16 % der Theorie) 5-(4-Chlorphenyl)-2-methyl-2-(pyrazol-1-yl)-3-(1,2,4-triazolmethyl)-pentan-3-ol vom Schmelzpunkt 118 °C.
Weitere 5 g können aus der bei der Umkristallisation verbleibenden Mutterlauge durch wiederholte Säulenchromatographie erhalten werden.

Herstellung von Ausgangsprodukten

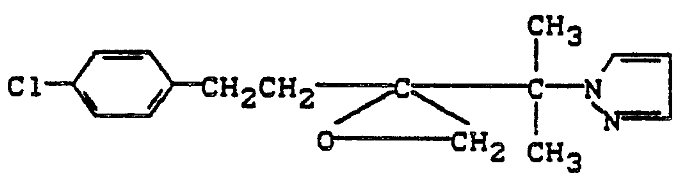

(II-1)

(Variante b)

17,6 g (0,08 Mol) Trimethylsulfoniumiodid werden in 50 ml Dimethylsulfoxid suspendiert. In das Gemisch werden 9 g (0,08 Mol) Kalium-tert-butylat innerhalb von 30 Minuten portionsweise unter Rühren bei Raumtemperatur eingetragen. Danach wird 1 Stunde bei Raumtemperatur nachgerührt. Anschließend wird bei 5 °C innerhalb einer Stunde ein Lösung von 22,1 g (0,08 Mol) 5-(4-Chlorphenyl)-2-methyl-2-(pyrazol-1-yl)-pentan-3-on in 20 ml Dimethylsulfoxid zugetropft. Nach 22-stündigem Rühren bei Raumtemperatur wird die Mischung in 1000 ml Wasser eingerührt. Man extrahiert dreimal mit 150 ml Methylenchlorid, trocknet die organische Phase und destilliert das Lösungsmittel im Wasserstrahlvakuum ab. Das erhaltene Oxiran wird ohne weitere Reinigung eingesetzt.

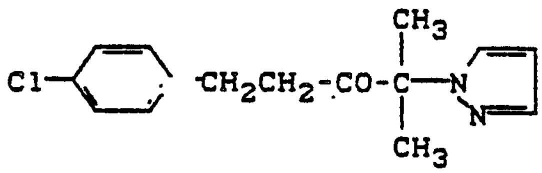

(IV-1)

In eine Lösung von 23,5 g (0,085 Mol) 5-(4-Chlorphenyl)-2-methyl-2-(pyrazol-1-yl)-pentan-3-ol in 60 ml Eisessig wird bei 5 °C innerhalb einer Stunde eine Lösung von 6,4 g (0,064 Mol) Chromsäure in 5 ml

Wasser und 20 ml Eisessig eingetropft. Es wird weitere 25 Stunden bei Raumtemperatur nachgerührt. Man rührt die Lösung in 2000 ml Wasser ein, extrahiert fünfmal mit je 200 ml Methylenchlorid und destilliert das Lösungsmittel ab. Man erhält 22,1 g (94 % der Theorie) 5-(4-Chlorphenyl)-2-methyl-2-(pyrazol-1-yl)-pentan-3-on vom Brechungsindex $n_D^{23}$ = 1,5278.

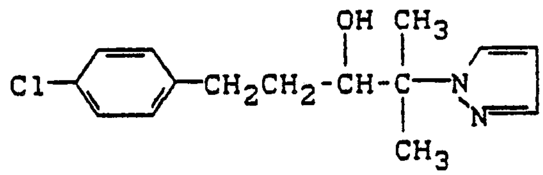

Eine Lösung von 27,4 g (0,1 Mol) 5-(4-Chlorphenyl)-2-methyl-2-(pyrazol-1-yl)-pent-3-on-4-en in 200 ml Methanol wird mit 7 g Raney-Nickel versetzt und 4 Stunden bei 60 °C mit Wasserstoff unter einem Druck von 40 bis 50 bar hydriert. Nach Absaugen des Katalysators und Abdestillieren des Methanols werden 27,2 g 5-(4-Chlorphenyl)-2-methyl-2-(pyrazol-1-yl)-pentan-3-ol vom Brechungsindex $n_D^{20}$ = 1,5438 erhalten.

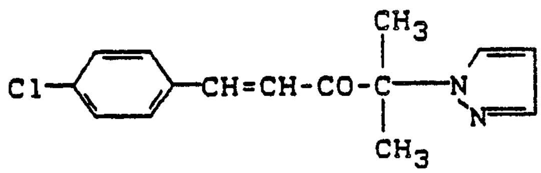

(IV-2)

In eine Lösung von 69,5 g (0,45 Mol) 2-Methyl-2-(pyrazol-1-yl)-butan-3-on in 200 ml Ethanol werden 44 ml Wasser sowie eine Lösung von 64 g (0,45 Mol) p-Chlorbenzaldehyd in 50 ml Ethanol gegeben. Dann tropft man eine Lösung von 1,35 g Natriumhydroxid in 13,5 ml Wasser hinzu. Die Reaktion ist exotherm bis 30 °C. Nach einer Stunde Nachrühren wird eine Lösung von 0,44 g (0,011 Mol) Natriumhydroxid in 4,4 ml Wasser zugetropft. Nach dreitägigem Stehen des Reaktionsgemisches bei Raumtemperatur wird das Lösungsmittel abdestilliert und der Rückstand in 400 ml Methylenchlorid aufgenommen. Die organische Phase wird dreimal mit je 300 ml Wasser und 10 ml Eisessig gewaschen. Die organische Phase wird unter vermindertem Druck eingeengt und der verbleibende Rückstand wird aus Petrolether umkristallisiert. Man erhält 91,6 g (74,2 % der Theorie) 5-(4-Chlorphenyl)-2 methyl-(pyrazol-1-yl)-pent-3-on-4-en vom Schmelzpunkt 60 °C.

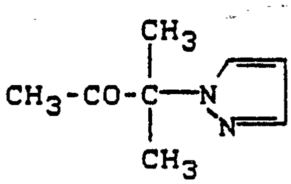

(XI-1)

Zu einer Schmelze von 4 Mol Pyrazol wird 1 Mol Methylbromisopropyl-keton bei 100 °C zugetropft. Das Reaktionsgemisch wird 1 Stunde nachgerührt. Nach üblicher Aufarbeitung erhält man 2-Methyl-2-(pyrazol-1-yl)-butan-3-on in einer Ausbeute von 70 %.

Beispiel 2

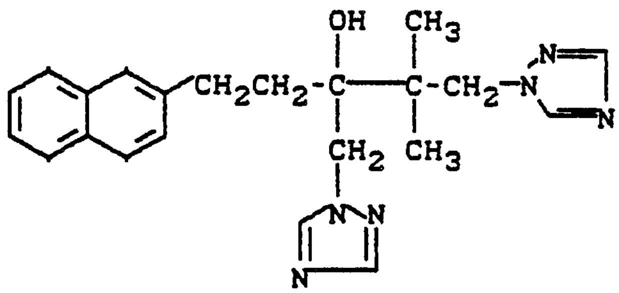

(I-2)

Eine Lösung von 10 g (0,031 Mol) 2-[1,1-Dimethyl-2-(1,2,4-triazol-1-yl)]-ethyl-2-(naphth-2-yl-ethyl)-oxiran, 2,4 g (0,0348 Mol) 1,2,4-Triazol, 0,4 g Natriumhydroxid, 0,1 g Wasser und einer Spatelspitze α,α'-Azoisobutyronitril in 100 ml Dimethylformamid wird 5 Stunden bei 100° C gerührt, anschließend abgekühlt und unter vermindertem Druck eingeengt. Der Rückstand wird in Dichlormethan gelöst. Die entstehende Lösung wird dreimal mit Wasser gewaschen, über Natriumsulfat getrocknet und unter vermindertem Druck eingeengt. Der Rückstand wird säulenchromatographisch (Kieselgel/Essigester und anschließend Essigester/Methanol = 1:1) gereinigt. Man erhält 6,0 g (49,6 % der Theorie) 4,4-Dimethyl-1-(2-naphthyl)-5-(1,2,4-triazol-1-yl)-3-(1,2,4-triazol-1-yl-methyl)-3-pentanol vom Schmelzpunkt 77-80° C.

Herstellung von Ausgangsprodukten

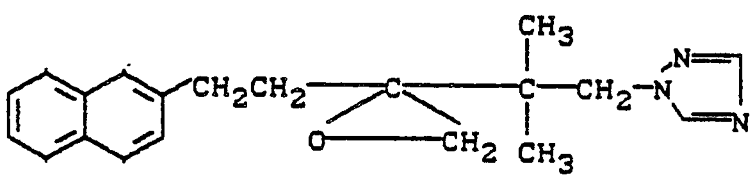

(II-2)

**(Variante a)**

Eine Lösung von 22 g (0,0721 Mol) 2-[1,1-Dimethyl-2-(1,2,4-triazol-1-yl)]-ethyl-4-(2-naphthyl)-1-buten in 50 ml Dichlormethan wird unter Rückfluß erhitzt. Innerhalb von 1,5 Stunden wird eine Lösung von 27,9 g (0,129 Mol) 80%-ige m-Chlorperbenzoesäure in 270 ml Dichlormethan eingetropft. Anschließend wird weitere 4 Stunden unter Rückfluß erhitzt, dann abgekühlt, dreimal mit 1-normaler Natronlauge und zweimal mit Wasser gewaschen. Die organische Phase wird über Natriumsulfat getrocknet und unter vermindertem Druck eingeengt. Es werden 19,0 g (82,1 % der Theorie) 2-[1,1-Dimethyl-2-(1,2,4-triazol-1-yl)]-ethyl-2-(naphth-2-yl-ethyl)-oxiran als Öl erhalten.

$^1$H-NMR (in $CDCl_3$: 300 MHz) $\delta$ = 2.60 (1H, d) und 2,73 (1H, d) für die Epoxid-Methylengruppe.

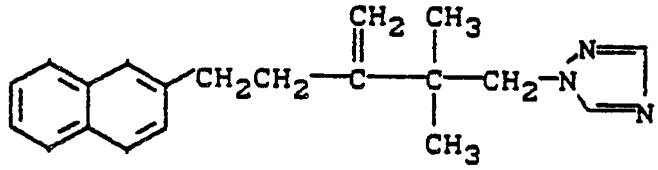

(VI-1)

Eine Suspension von 57,3 g (0,16 Mol) Methyltriphenylphosphoniumbromid und 18,4 g (0,16) Mol) Kalium-tert-butylat in 250 ml absolutem Toluol wird unter trockenem Stickstoff auf 90° C erwärmt. Innerhalb von 30 Minuten wird eine Lösung von 36,8 g (0,12 Mol) 4,4-Dimethyl-1-(2-naphthyl)-5-(1,2,4-triazol-1-yl)-3-pentanon in 200 ml absolutem Toluol eingetropft. Das Reaktionsgemisch wird anschließend 2 Stunden bei 90° C nachgerührt, abgekühlt, zweimal mit wasser gewaschen und unter vermindertem Druck eingeengt.

Der Rückstand wird in Essigester aufgenommen, auf 5° C gekühlt und der dabei gebildete Kristallbrei (Triphenylphosphoniumoxid) abfiltriert. Das Filtrat wird unter vermindertem Druck eingeengt, und der Rückstand mittels Säulenchromatographie gereinigt (Kieselgel; Dichlormethan/Essigester = 9:1).

Man erhält 27,0 g (73,8 % der Theorie) 2-[1,1-Dimethyl-2-(1,2,4-triazol-1-yl)-ethyl-4-(2-naphthyl)-1-buten in Form eines öligen Produktes.

$^1$H-NMR (300 MHz $CDCl_3$):$\delta$ = 4,91 (1H, s) und 5,05 (1H, s) für die olefiniche Methylengruppe.

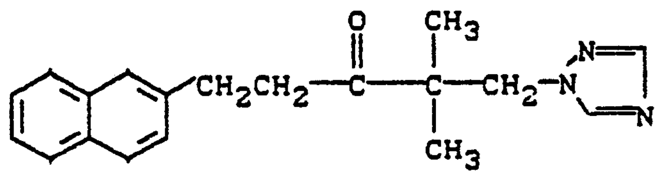

(IV-3)

Eine Lösung von 67 g (0,22 Mol) 4,4-Dimethyl-1-(2-naphthyl)-5-(1,2,4-triazol-1-yl)-1-penten-3-on in 400 ml Methanol wird mit 15 g Raney-Nickel versetzt und im Autoklaven 4 Stunden bei 90° C unter 90 bar Wasserstoff gerührt. Nach Filtrieren und Einengen des Reaktionsgemisches unter vermindertem Druck erhält man 55 g (81,4 % der Theorie) 4,4-Dimethyl-1-(2-naphthyl)-5-(1,2,4-triazol-1-yl)-3-pentanon als gelben Feststoff vom Schmelzpunkt 134° C.

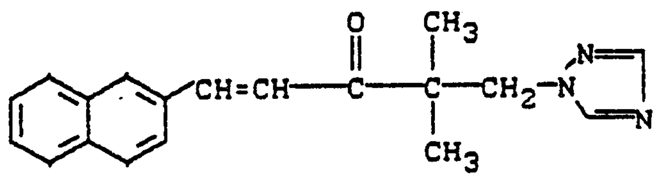

(IV-4)

59,3 g (0,38 Mol) 2-Naphthaldehyd und 63,5 g (0,38 Mol) 3,3-Dimethyl-4-(1,2,4-triazol-1-yl)-2-butanon werden in 200 ml Ethanol gelöst und mit einer Lösung von 1,5 g Natriumhydroxid in 15 ml Wasser versetzt. Das Gemisch wird 1 Stunde bei Raumtemperatur gerührt, mit 0,5 g festem Natriumhydroxid versetzt, 1 Stunde weitergerührt, mit 50 ml Wasser versetzt und weitere 16 Stunden gerührt. Das Reaktionsgemisch wird dann mit 100 ml Wasser versetzt. Der Niederschlag wird abgesaugt und in Di chlormethan gelöst. Die Lösung wird zweimal mit Wasser gewaschen, über Natriumsulfat getrocknet und unter vermindertem Druck eingeengt.

Man erhält 115 g (99 % der Theorie) 4,4-Dimethyl-1-(2-naphthyl)-5-(1,2,4-triazol-1-yl)-1-penten-3-on als weißen Feststoff vom Schmelzpunkt 103-104° C.

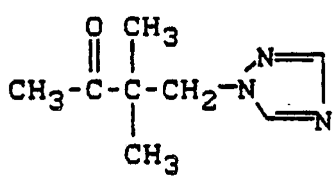

(XI-2)

In eine Lösung von 138 g (2 Mol) 1,2,4-Triazol in 1600 ml absolutem Dimethylformamid werden 60 g (2 Mol) Natriumhydrid (80 %-ig in Paraffinöl) innerhalb von 3 Stunden eingetragen, wobei Wasserstoff kräftig entweicht. Das Gemisch wird 30 Minuten bei Raumtemperatur nachgerührt. Danach werden 388 g (2 Mol) 3,3-Dimethyl-4-[(methylsulfonyl)oxy]-2-butanon innerhalb von 15 Minuten eingetropft. Dann wird das Reaktionsgemisch 16 Stunden bei 95° C gerührt. Das Reaktionsgemisch wird zunächst vorsichtig mit 100 ml Wasser versetzt und anschließend in 4 l Wasser gegossen. Man extrahiert sechsmal mit Dichlormethan. Die vereinigten organischen Phasen werden einmal mit viel Wasser gewaschen, über Natriumsulfat getrocknet und unter Hochvakuum eingeengt. Nach Säulenchromatographie (Kieselgel; Diohlormethan/Essigester = 1:1) erhält man 290 g (86,8 % der Theorie) 3,3-Dimethyl-4-(1,2,4-triazol-1-yl)-2-butanon als Öl mit einem Gehalt von 97 % nach GC.

$^1$H-NMR (300 MHz $CDCl_3$): $\delta$ = 1,23 (6H, s; $C(CH_3)_2$, 2,19 (3H, s; $CH_3CO$), 4,32 (2H, s; $CH_2$-Triazol), 7,90 (1H, s) und 8,12 (1H, s) für die Triazol-Ringprotonen.

Entsprechend den Herstellungsbeispielen 1 und 2 sowie den erfindungsgemäßen Verfahrensangaben werden die nachfolgenden Verbindungen der Formel (I) erhalten:

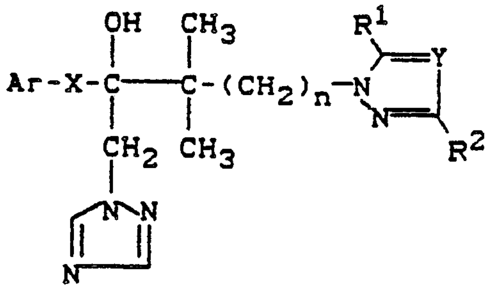

(I)

| Bsp. Nr. | Ar | X | n | 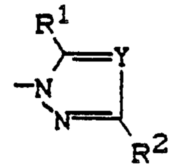 | Fp(°C) |
|---|---|---|---|---|---|
| 3 | 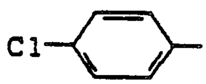 | $-CH_2CH_2-$ | 1 | 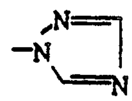 | Harz |
| 4 | 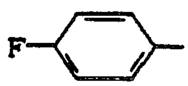 | $-CH_2CH_2-$ | 1 | 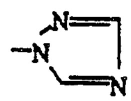 | zähes Öl |
| 5 | 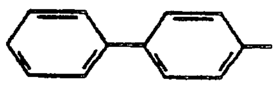 | $-CH_2CH_2-$ | 1 | 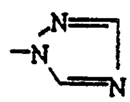 | 96-98 |
| 6 | 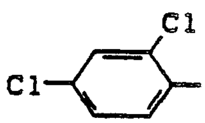 | $-CH_2CH_2-$ | 1 | 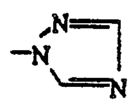 | 116-117 |
| 7 | 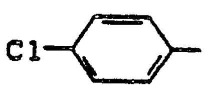 | -CH=CH- | 0 | 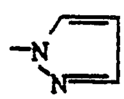 | 132 (trans-Isomeres) |
| 8 | 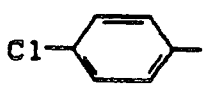 | -CH=CH- | 0 | 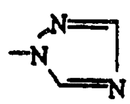 | 198 |

| Bsp. Nr. | Ar | X | n | 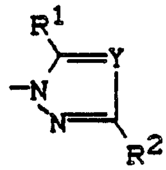 | Fp(°C) |
|---|---|---|---|---|---|
| 9 | 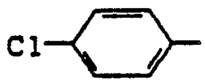 | $-SCH_2-$ | 0 | 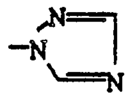 | 92 |
| 10 | 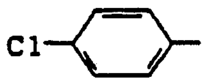 | $-OCH_2-$ | 0 | 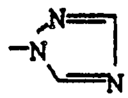 | 95 |
| 11 | 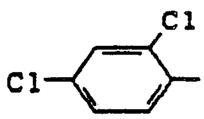 | $-OCH_2-$ | 0 | 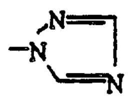 | 140 |
| 12 | 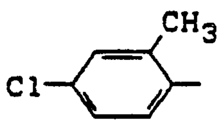 | $-OCH_2-$ | 0 | 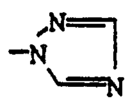 | 154 |
| 13 | 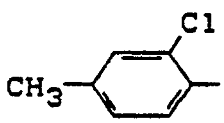 | $-OCH_2-$ | 0 | 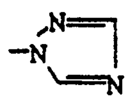 | 129 |
| 14 | 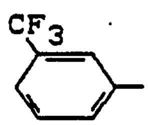 | $-OCH_2-$ | 0 | 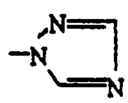 | 122 |
| 15 | 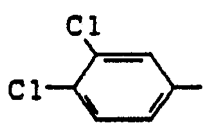 | $-OCH_2-$ | 0 | 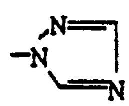 | 150 |
| 16 | 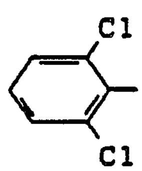 | $-CH_2CH_2-$ | 1 | 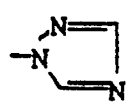 | 82-86 |

| Bsp. Nr. | Ar | X | n | 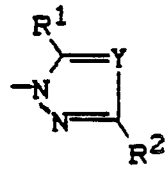 | Fp(°C) |
|---|---|---|---|---|---|
| 17 | 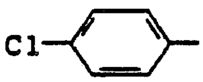 | $-CH_2CH_2-$ | 0 | 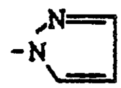 | -50 (x HCl) (hygroskopisch) |
| 18 | 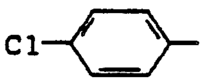 | $-CH_2CH_2-$ | 0 | 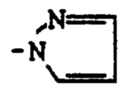 | 110 [x 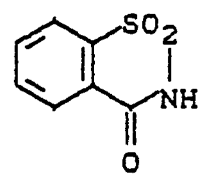 |
| 19 | 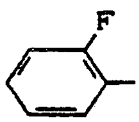 | $-CH_2CH_2-$ | 0 | 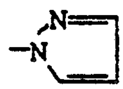 | -35 |
| 20 | 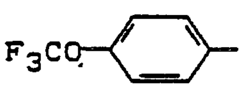 | $-CH_2CH_2-$ | 0 | 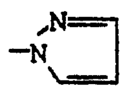 | 92 |
| 21 | 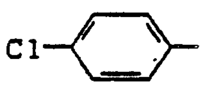 | $-CH_2CH_2-$ | 0 | 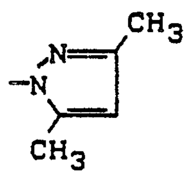 | Öl |
| 22 | 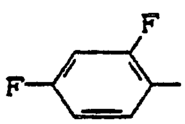 | $-CH_2CH_2-$ | 0 | 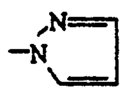 | 104 |
| 23 | 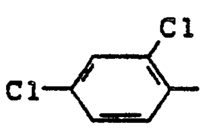 | $-CH_2CH_2-$ | 0 | 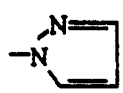 | 84 |
| 24 | 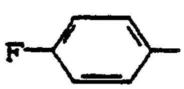 | $-CH_2CH_2-$ | 0 | 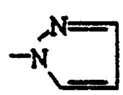 | 120 |

| Bsp. Nr. | Ar | X | n | 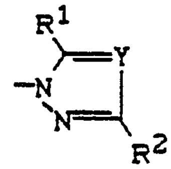 | Fp(°C) bzw. Berechnungsindex ($n_D20$) |
|---|---|---|---|---|---|
| 25 | 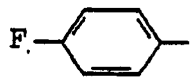 | $-CH_2CH_2-$ | 0 | 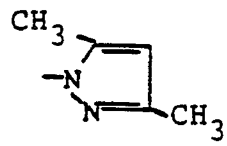 | $n_D^{20}$ = 1,5394 |
| 26 | 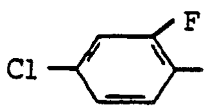 | $-CH_2CH_2-$ | 0 | 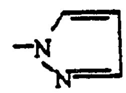 | 102 |
| 27 | 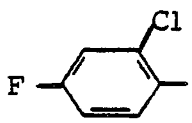 | $-CH_2CH_2-$ | 0 | 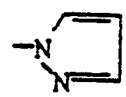 | $n_D^{20}$ = 1,5399 |
| 28 | 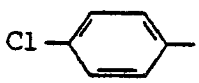 | $-OCH_2-$ | 0 | 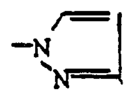 | 146 |
| 29 | 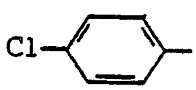 | $-CH_2CH_2-$ | 0 | 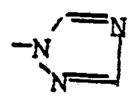 | zähfl. Öl |
| 30 | 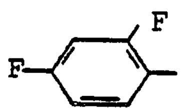 | $-CH_2CH_2-$ | 1 | 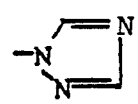 | 109 |

In den folgenden Verwendungsbeispielen werden die nachstehend angegebenen Verbindungen als Vergleichssubstanzen eingesetzt:

(A) 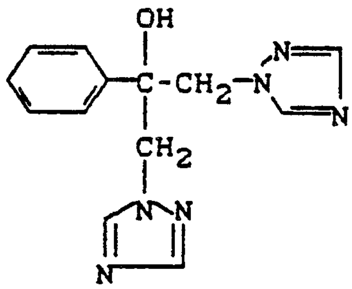

(bekannt aus EP-OS 0 044 605)

(B) 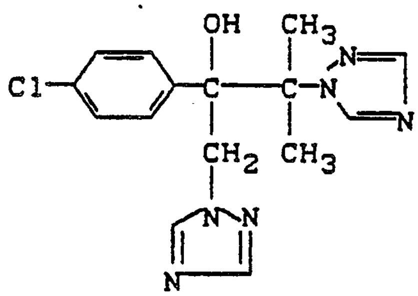

(C) 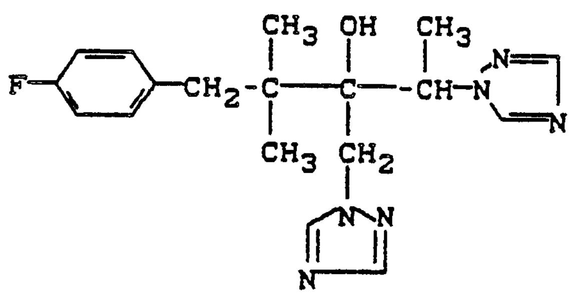

(D) 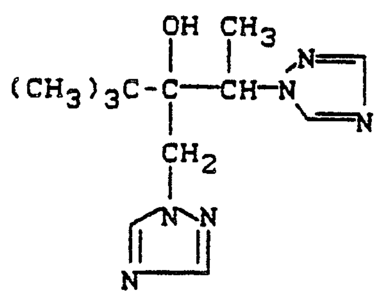

(bekannt aus EP-OS 0 131 845)

Beispiel A

Erysiphe-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigen die in den Beispielen 2, 4, 7, 8, 10, 12 und 15 beschriebenen erfindungsgemäßen Stoffe eine wesentlich bessere Wirksamkeit als die Vergleichssubstanz (B).

Beispiel B

Sphaerotheca-Test (Gurke)/protektiv

Lösungsmittel 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

In diesem Test zeigt die in dem Beispiel 10 beschriebene erfindungsgemäße Substanz eine wesentlich bessere Wirksamkeit als die Vergleichssubstanzen (A), (B) und (C).

Beispiel C

Venturia-Test (Apfel)/kurativ

Lösungsmittel: 0,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20 °C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

12 Tage nach der Inokulation erfolgt die Auswertung.

In diesem Test zeigen die in den Beispielen 7 und 15 beschriebenen erfindungsgemäßen Stoffe eine wesentlich bessere Wirksamkeit als die Vergleichssubstanzen (C) und (D).

## Ansprüche

1. Bisazolyl-hydroxyalkyl-Derivate der Formel

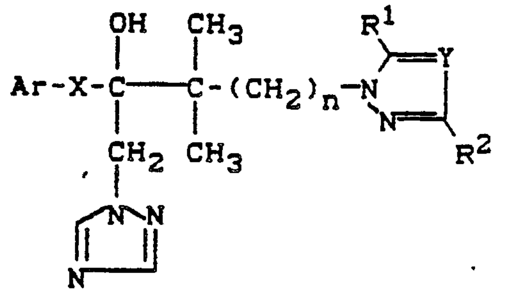 (I)

in welcher
Ar für gegebenenfalls substituiertes Aryl steht,
X für eine Gruppierung $-CH_2CH_2-$, $-OCH_2-$, $-SCH_2-$oder $-CH=CH-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht,
sowie deren Säureadditions-Salze und Metallsalz-Komplexe.

2. Bisazolyl-hydroxyalkyl-Derivate der Formel (I) gemäß Anspruch 1, in denen
Ar für Phenyl steht, das einfach bis dreifach, gleichartig oder verschieden substituiert sein kann durch Halogen, Alkyl mit 1 bis 4 Kohlenstoffatomen, Alkoxy und Alkylthio mit jeweils 1 oder 2 Kohlenstoffatomen, Nitro, Halogenalkyl, Halogenalkoxy und Halogenalkylthio mit jeweils 1 oder 2 Kohlenstoffatomen und 1 bis 5 gleichen oder verschiedenen Halogenatomen, Alkoximinoalkyl mit 1 bis 4 Kohlenstoffatomen im Alkoxyteil und 1 oder 2 Kohlenstoffatomen im Alkylteil sowie jeweils gegebenenfalls durch Halogen und/oder Alkyl mit 1 oder 2 Kohlenstoffatomen substituiertes Phenyl, Phenoxy, Benzyl und/oder Benzyloxy, oder
Ar für Naphthyl steht, das einfach bis dreifach, gleichartig oder verschieden substituiert sein kann durch Halogen, Alkyl mit 1 oder 2 Kohlenstoffatomen, Alkoxy mit 1 oder 2 Kohlenstoffatomen und/oder Halogenalkoxy mit 1 oder 2 Kohlenstoffatomen und 1 bis 5 gleichen oder verschiedenen Halogenatomen,
X für die Gruppierungen $-CH_2CH_2-$, $-OCH_2-$, $-SCH_2-$oder $-CH=CH-$ steht, wobei das Heteroatom mit dem Aryl-Rest verbunden ist, wenn X für $-OCH_2-$oder $-SCH_2-$steht;
Y für ein Stickstoffatom oder die Gruppierung $CR^3$ steht;
$R^1$, $R^2$ und $R^3$ für Wasserstoff, Chlor oder Methyl stehen und
n für die Zahlen 0, 1 oder 2 steht.

3. Verfahren zur Herstellung von Bisazolyl-hydroxyalkyl-Derivaten der Formel

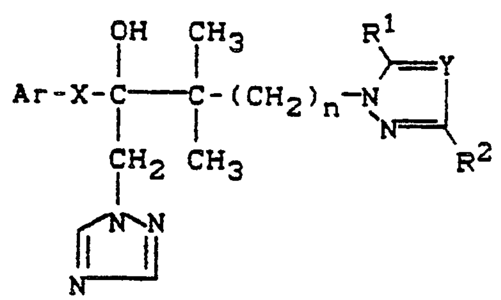 (I)

in welcher
Ar für gegebenenfalls substituiertes Aryl steht,
X für eine Gruppierung $-CH_2CH_2-$, $-OCH_2-$, $-SCH_2-$oder $-CH=CH-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht,
sowie von deren Säureadditions-Salzen und Metallsalz-Komplexen, dadurch gekennzeichnet, daß man

Oxirane der Formel

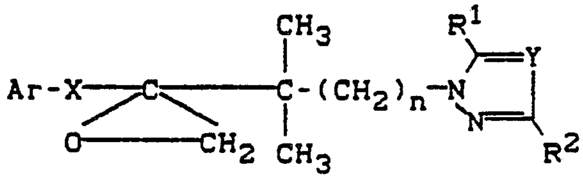 (II)

in welcher
Ar, X, Y, $R^1$, $R^2$ und n die oben angegebene Bedeutung haben,
mit 1,2,4-Triazol der Formel

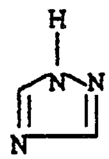 (III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

4. Fungizide Mittel, gekennzeichnet durch einen Gehalt an midestens einem Bisazolyl-hydroxyalkyl-Derivat der Formel (I) gemäß Anspruch 1 bzw. an einem Säureadditions-Salz oder Metallsalz-Komplex eines Bisazolyl-hydroxyalkyl-Derivates der Formel (I).

5. Verwendung von Bisazolyl-hydroxyalkyl-Derivaten der Formel (I) gemäß Anspruch 1 bzw. von deren Säureadditions-Salzen und Metallsalz-Komplexen zur Bekämpfung von Pilzen.

6. Oxirane der Formel

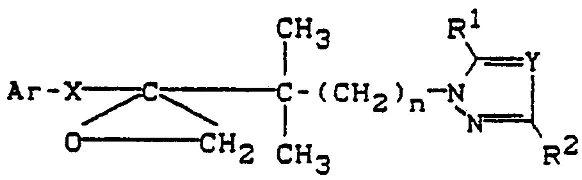 (II)

in welcher
Ar für gegebenenfalls substituiertes Aryl steht,
X für eine Gruppierung $-CH_2CH_2-$, $-OCH_2-$, $-SCH_2-$oder $-CH=CH-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht.

7. Verfahren zur Herstellung von Oxiranen der Formel

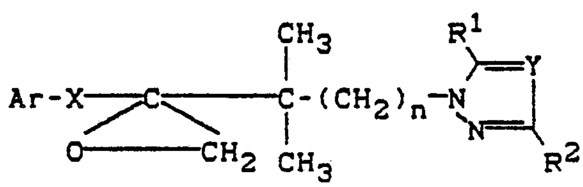 (II)

in welcher
Ar für gegebenenfalls substituiertes Aryl steht,
X für eine Gruppierung $-CH_2CH_2-$, $-OCH_2-$, $-SCH_2-$oder $-CH=CH-$ steht,

Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht,
dadurch gekennzeichnet, daß man

a) in einer ersten Stufe Ketone der Formel

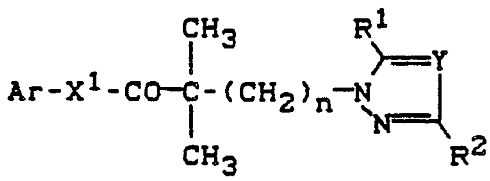

(IVa)

in welcher
Ar, Y, $R^1$, $R^2$ und n die oben angegebene Bedeutung haben und
$X^1$ für die Gruppierungen $-CH_2CH_2-$, $-OCH_2-$und $-SCH_2-$ steht,
mit Methyl-triphenyl-phosphoniumbromid der Formel

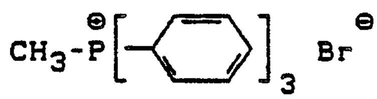

(V)

in Gegenwart einer Base und in Gegenwart eines Verdünnungsmittels umsetzt und dann in einer zweiten Stufe die so erhaltenen Verbindungen der Formel

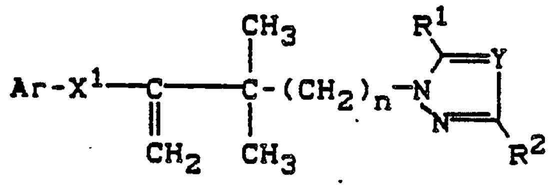

(VI)

in welcher
Ar, $X^1$, Y, $R^1$, $R^2$ und n die oben angegebene Bedeutung haben,
mit Persäure in Gegenwart eines Verdünnungsmittels umsetzt;
oder

(b) Ketone der Formel

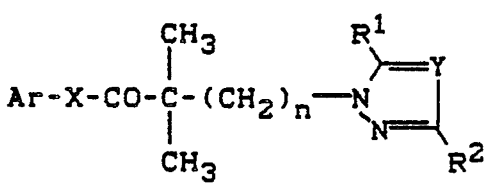

(IV)

in welcher
Ar, X, Y, $R^1$, $R^2$ und n die oben angegebene Bedeutung haben,
entweder
α)mit Dimethyloxosulfonium-methylid der Formel

$$\overset{\delta\oplus}{(CH_3)_2SO}\ \overset{\delta\ominus}{CH_2} \qquad (VII)$$

oder
β) mit Dimethylsulfonium-methylid der Formel

$$(CH_3)_2\overset{\delta\ominus}{S}\;\overset{\delta\ominus}{C}H_2 \qquad (VIII)$$

in Gegenwart eines Verdünnungsmittels umsetzt

8. Keton der Formel

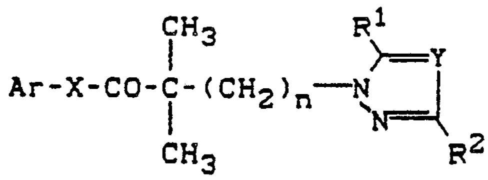

(IV)

Ar für gegebenenfalls substituiertes Aryl steht,
X für eine Gruppierung $-CH_2CH_2-$, $-OCH_2-$, $-SCH_2-$oder $-CH=CH-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht.

9. Verfahren zur Herstellung von Ketonen der Formel

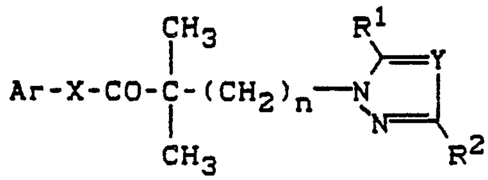

(IV)

Ar für gegebenenfalls substituiertes Aryl steht,
X für eine Gruppierung $-CH_2CH_2-$, $-OCH_2-$, $-SCH_2-$oder $-CH=CH-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht,
dadurch gekennzeichnet, daß man entweder Halogenketone der Formel

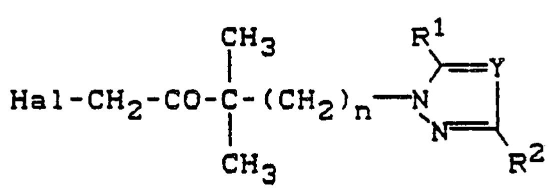

(IX)

in welcher
Y, $R^1$, $R^2$ und n die oben angegebene Bedeutung haben und
Hal für Chlor oder Brom steht,
mit Verbindungen der Formel

Ar-Z-H (X)

in welcher
Ar die oben angegebene Bedeutung hat und
Z für Sauerstoff oder Schwefel steht,
in Gegenwart einer Base und in Gegenwart eines Verdünnungsmittels umsetzt,
oder

Verbindungen der Formel

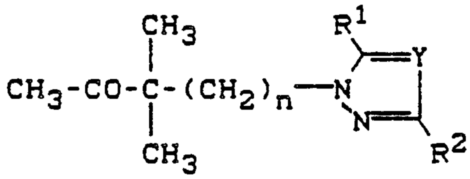

(XI)

in welcher
Y, $R^1$, $R^2$ und n die oben angegebene Bedeutung haben
mit Aldehyden der Formel,

Ar-CHO (XII)

in welcher
Ar die oben angegebene Bedeutung hat,
in Gegenwart eines Verdünnungsmittels und in Gegenwart einer Base umsetzt und gegebenenfalls die so erhaltenen Verbindungen der Formel

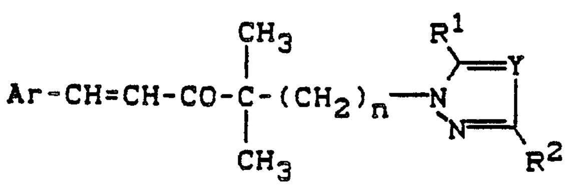

(IVd)

in welcher
Ar, $R^1$, $R^2$, Y und n die oben angegebene Bedeutung haben,
mit Wasserstoff in Gegenwart eines Katalysators und in Gegenwart eines Verdünnungsmittels umsetzt.

10. Verbindungen der Formel

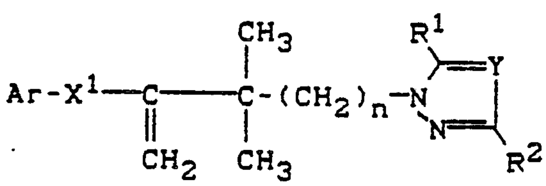

(VI)

in welcher
Ar für gegebenenfalls substituiertes Aryl steht,
$X^1$ für einer Gruppierung $-CH_2CH_2-$, $-OCH_2-$, oder $-SCH_2-$ steht,
Y für ein Stickstoffatom oder die Gruppierung $CR^3$ steht,
$R^1$, $R^2$ und $R^3$ für Wasserstoff, Halogen oder Methyl stehen und
n für die Zahlen 0, 1 oder 2 steht.

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

Nummer der Anmeldung

EP 88114655.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2/A3 - 0 131 845 (BAYER) * Formeln I,II,IV * -- | 1,6,8 | C 07 D 249/08<br>C 07 D 233/60<br>A 01 N 43/653<br>A 01 N 43/50 |
| A | EP - A1 - 0 091 398 (CIBA-GEIGY) * Formeln Ia,I,II * -- | 1,6 | |
| A | EP - A2 - 0 180 838 (BAYER) * Zusammenfassung; Formeln II, IV * -- | 1,6,8 | |
| A | EP - A2 - 0 207 590 (JCJ) * Anspruch 1; Formeln IIa, IV * -- | 1,6,8 | |
| A | EP - A1 - 0 105 166 (BAYER) * Formeln I,V * ---- | 1,6 | |

RECHERCHIERTE SACHGEBIETE (Int. Cl.4)

C 07 D 249/00
C 07 D 233/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4
Unvollständig recherchierte Patentansprüche: 5
Nicht recherchierte Patentansprüche: -
Grund für die Beschränkung der Recherche: Soweit es einVerfahren zur therapeutischen Behandlung des menschlichen oder tierischen Körpers betrifft (EPÜ, Art. 52(4))

| Recherchenort | Abschlußdatum der Recherche | Prüfer |
|---|---|---|
| WIEN | 27-10-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument